# EUROPEAN PATENT SPECIFICATION

(11) **EP 2 638 169 B1**
(45) Date of publication and mention of the grant of the patent: **07.01.2015**
(21) Application number: 11779387.7
(22) Date of filing: 02.11.2011
(51) Int. Cl.: C12P 5/02, C12P 7/06, C12M 1/04, C07C 1/24, C07C 11/04

(54) **PROCESS AND APPARATUS FOR PRODUCING ETHYLENE VIA PREPARATION OF SYNGAS**
VERFAHREN UND VORRICHTUNG ZUR PRODUKTION VON ETHYLEN VIA HERSTELLUNG VON SYNTHESEGAS
PROCÉDÉ ET INSTALLATION DE PRODUCTION DE L'ÉTHYLÈNE PAR PRÉPARATION DE SYNGAS

(30) Priority: 09.11.2010 EP 10190539
(43) Date of publication of application: 18.09.2013
(73) Proprietor: Ineos Sales (UK) Limited, Lyndhurst Hampshire SO43 7FG (GB)
(72) Inventor: BELL, Peter Simpson, Dunblane FK15 9BB (GB); PARKER, Graeme Alexander, Dunmore Falkirk FK2 8LX (GB); TURNBULL, Neil, Dalgety Bay Fife KY11 9UB (GB); WILLIAMS, Vaughan Clifford, Edinburgh Midlothian EH11 1QU (GB)
(74) Representative: Hartley, Andrew Philip
(86) International application number: PCT/EP2011/069237
(87) International publication number: WO 2012/062633

(56) References cited:
- WO-A2-2009/010347
- WO-A2-2010/012946
- US-A1- 2010 294 642
- SCHULZ, J. & BANDERMANN, F.: "Conversion of Ethanol over Zeolite H-ZSM-5", CHEMICAL ENGINEERING AND TECHNOLOGY, vol. 17, no. 3, June 1994 (1994-06), pages 179-186, XP002648266,

## Description

### BACKGROUND OF THE INVENTION

### 1. Field Of The Invention

This invention relates to processes and apparatus for producing olefin derivatives. More especially the invention relates to the production of olefin derivatives such as ethylene, polyethylene, ethylbenzene, ethylene oxide, ethylene glycol, ethanolamine, crown ethers and vinyl chloride monomer via bacterial fermentation.

Many olefin derivatives are produced from oil. It is desirable to be able to produce them from a wider range of starting materials.

### 2. Description Of The Prior Art

WO2010/012 946 (Arkema) describes a process for making polyethylene from biomass. The biomass is fermented to convert cellulose, sugars and starches into ethanol using the yeast *Saccharomyces cerivisiae.* The ethanol is dehydrated to produce a dilute solution of ethanol in water which is concentrated. The ethanol is then dehydrated to produce ethylene which is polymerised. As explained in WO2010/012 946 the process is suitable for small scale plants not large scale industrial processes.

An aspect of the invention seeks to overcome the problems with the prior art and provide a large-scale process for producing ethylene and olefin derivatives via a fermentation step.

Processes are known for producing ethylene and olefin derivatives from methane as will be described hereinafter but the yield of ethylene is low. The invention seeks to produce ethylene in high yield.

### SUMMARY OF THE INVENTION

According to the invention there is provided a process for producing ethylene from a carbonaceous containing feedstock the process comprising the steps of
a) reforming the carbonaceous containing feedstock into a first product stream comprising carbon dioxide, carbon monoxide and hydrogen;
b) fermenting the first product stream with an acetogenic anaerobic bacteria to produce a second product stream comprising ethanol;
c) concentrating ethanol from the second product stream into a third product stream; and
d) dehydrating ethanol in the third product stream to produce a first, ethylene enriched, carbonaceous fraction, a second, ethylene depleted, carbonaceous fraction and water and
d1) recycling at least a part of the water, optionally after treatment, to step b) and/or to step a) and/or recycling at least a part of the second, ethylene depleted carbonaceous fraction as a feedstock to step a).

According to the invention there is further provided apparatus for producing ethylene comprising
i) a reformer for reforming a carbonaceous feedstock into a first product stream comprising carbon dioxide, carbon monoxide and hydrogen;
ii) a fermenter for fermenting the first product stream into a second product stream comprising ethanol;
iii) means for concentrating ethanol from the second product stream;
iv) a dehydrator for dehydrating the ethanol to a first, ethylene enriched, carbonaceous fraction, a second, ethylene depleted, carbonaceous fraction and water; and either or both
v) means for recycling at least a portion of the second, ethylene depleted, carbonaceous fraction to the reformer and
vi) means for recycling at least a portion of the water to the fermenter and/or reformer.

In outline a carbonaceous feedstock such as natural gas, shale gas, coal mine methane, biogas (such as that derived from, for example, the anaerobic digestion of biomass or alternatively from wastes, often also known as landfill gas), carbonaceous gas hydrates or clathrates, associated gas and refinery gas, is converted to syngas. Syngas is a mixture of hydrogen, carbon monoxide and carbon dioxide.

The syngas is then subjected to a bacterial fermentation to produce, principally, ethanol together with some minor by-products. Typical microorganisms utilised are acetogenic anaerobic bacteria especially such clostridium species as *Clostridium ljungdahlii, C. carboxydivorans, C. ragsdalei* and *C*. *autoethanogenum.*

The ethanol is then dehydrated, preferably catalytically, into ethylene.

The ethylene is then converted into an olefin derivative. Examples include solids such as polyethylene, liquids such as ethylbenzene or gases such as ethylene oxide. The olefin derivative may itself be further transformed for example in the case of ethylbenzene to styrene and then to polystyrene. Ethylene oxide can be transformed into ethylene glycol, ethanolamine, crown ethers and other materials which can again be subject to further transformation.

It has now surprisingly been found that by conducting the fermentation step in the same location as (i.e. within 5km, preferably within 4km, 3km, 2km or 1km of) the dehydration step and preferably at least one conversion step in the same location as the fermentation step (ie within 5km, preferably within 4km, 3km, 2km or 1km of) unexpected benefits accrue especially in large scale plants. In particular in view of the differing energy, water and other chemical component requirements of each stage of the overall process it is possible to recycle by-product and energy from one stage to another stage resulting in overall greater efficiency. By using acetogenic anaerobic bacteria fermenting syn-gas it is possible to convert relatively inexpensive raw materials into olefin derivatives on a large scale.

### DETAILED DESCRIPTION OF THE INVENTION

The process of the invention has material advantages over situations where the fermentation step is not co-located with the dehydration and ethylene derivative process. Where units are not co-located, such as for lack of local ethylene derivative market, product ethanol (defined herein as the third product stream) would need to be transported in bulk by road, rail or ship to a more suitable location for dehydration and ethylene conversion. Significant stream integration benefits would be lost, however the advantages described hereinafter over other routes from carbonaceous feedstock to ethylene and ethylene derivatives would remain. As an initial step syngas is prepared from a carbonaceous feedstock. Any suitable feedstock can be used. In the case of biomass feedstock being utilised several processes are known for converting such feeds into a methane containing biogas, one such well documented process is anaerobic digestion but others including pyrolysis could produce a gas suitable to be reformed to syngas. In addition biogas derived from waste disposal, known as landfill gas can be utilised by the process of the invention. In an embodiment of the invention biomass is converted into methane containing biogas by anaerobic digestion where the energy to maintain such a bacterial system at its optimal temperature usually above ambient conditions may come from heat evolved by exothermic steps elsewhere in the process as described hereinafter. The resulting biogas is then converted into syngas in known manner by reforming. In general terms however biomass is not a preferred carbonaceous feedstock because it is not easily and cheaply available in large enough volumes for large-scale use. Additionally conversion of biomass to biogas can be inefficient in that few bacteria convert lignin into useful materials and there are thus large quantities of only partially digested wet feedstock to be disposed of.

In preferred embodiments the feedstock contains a substantial amount of methane especially fossil methane.

The feedstock is fed to at least one reformer where it is converted to a mixture of hydrogen, carbon dioxide and carbon monoxide. A number of reforming processes are known. They include
1) steam methane reforming (SMR) in which the methane containing feedstock is reformed in an externally fired reformer in the presence of >2:1, preferably >2.5:1 molar steam : methane ratio.
2) autothermal reforming (ATR) in which the methane containing feedstock is reformed in the presence of steam and oxygen
3) partial oxidation (POX) in which the methane containing feedstock is reformed in the presence of oxygen and relatively low or zero concentrations of steam and
4) dry reforming in which the methane containing feedstock is reformed in an externally fired reformer in the presence of carbon dioxide and low or zero concentrations of feed steam.

A preferred reforming process is sulphur passivated reforming (SPARG) which is described in Hydrocarbon Processing, January 1986 p 71-74 and Oil and Gas Journal Mar 9, 1992, p 62-67. In a SPARG process sulphur is added to passivate the reforming catalyst. The sulphur reduces cokes formation, which can otherwise be a problem. It has been reported that the sulphur blocks large sites on the catalyst which are required to form coke but leaves the small sites open allowing reforming to continue.

The SPARG process is thought not to be widely used in the production of syngas. Reasons for this may include that most processes which use syngas require higher H₂:CO ratios than are produced by SPARG reforming and because sulphur is generally a catalyst poison and thus needs to be removed from the syngas before it is used in many applications. Bacterial fermentations producing ethanol from syngas are generally tolerant of sulphur and so catalyst poisoning is not therefore a problem.

The different reforming processes give rise to a different mix of products with an SMR having an H₂: CO molar ratio in the range of about 3-5:1 compared with 2:1 for an ATR and 1.7 to 1.8:1 for a POX. An SMR typically produces syngas having a CO₂:CO molar ratio of 0.35:1 compared with 0.2:1 for an ATR and <0.1:1 for a POX. Dry gas reforming produces a feed gas with a reduced H₂:CO ratio and more effective conversion of CO₂ to CO than an SMR.

In some embodiments of the invention a plurality of reformers are used. In some embodiments of the invention the reformers are placed in series with the output from a first reactor optionally after treatment forming at least part of the input of a second reactor of the same or different type. An example of this is the so-called Lurgi Combined Reforming Process in which the methane containing feedstock, any recycled gases are fed to an SMR along with steam. The output from this reformer together, optionally, with unreacted feedstock and/or recycled gases is fed to an ATR. Syngas can be produced in a process in which initial feedstock optionally with recycle gases is first fed to an SMR, ATR or POX reformer. The output from that reformer optionally with unreacted feedstock and/or recycle gases is fed with steam to an ATR or POX reformer. By using a plurality of reactors in series the composition of the syngas can be different from that obtained from a single reactor and more suitable for further processing. In some embodiments the syngas is produced in processes in which the feedstock and optionally recycle gas is fed to a plurality of reactors which may be the same or different which are at least partially arranged in parallel. In addition to allowing the product mix to be different from that obtained when a single reformer is used the arrangement produces redundancy. In the event that one reformer fails, the redundancy allows syngas to continue to be produced. The culture used in the fermentation can thus be kept alive reducing or eliminating process plant down time.

The syngas exiting the reformers is cooled in one or more heat exchangers. The abstracted heat can be either transferred, in the form of steam, to other parts of the process or by direct exchange with streams from other parts of the overall process requiring to be heated by way of example the heat could be passed directly or by transfer of steam to the distillation reboilers to be described hereinafter. Our cofiled application claiming priority from EP 10 190 527.1 (now published as WO2012062631) describes such a process in more detail.

In a most preferred embodiment, the process of the present invention is operated at elevated pressure in both the reforming and fermentation steps. Preferably the pressure is in the range 2 to 20 barg for both steps. The pressure is preferably based on the optimum pressure for the fermentation step. The reforming process is operated at a suitably higher pressure to allow for inherent pressure loss between process steps as outlined previously, to provide the product stream at the required pressure for the fermentation step, and with minimal compression required for the recycle of the fourth gaseous product stream to the reforming process. One further advantage of SPARG technology, for example, is that it may be operated across a wide range of pressures dependent on the downstream processing required without significant variations in product distribution.

The fermentation process may use any suitable bacteria. The preferred fermentation process uses acetogenic anaerobic bacteria, especially a rod-shaped, gram positive, non-thermophilic anaerobe. Examples of useful acetogenic bacteria include those of the genus *Clostridium,* such as strains of *Clostridium ljungdahlii,* including those described in WO 2000/68407, EP 117309, U.S. Patent Nos. 5,173,429, 5,593,886 and 6,368,819, WO 1998/00558 and WO 2002/08438, strains of *Clostridium autoethanogenum* (DSM 10061 and DSM 19630 of DSMZ, Germany) including those described in WO 2007/117157 and WO 2009/151342 and *Clostridium ragsdalei* (P11, ATCC BAA-622) and *Alkalibaculum bacchi* (CP11, ATCC BAA-1772) including those described respectively in U.S. Patent No. 7,704,723 and "Biofuels and Bioproducts from Biomass-Generated Synthesis Gas", Hasan Atiyeh, presented in Oklahoma EPSCoR Annual State Conference, April 29, 2010 and *Clostridium carboxidivorans* (ATCC PTA-7827) described in U.S. Patent Application No. 2007/0276447. Other suitable microorganisms includes those of the genus *Moorella,* including *Moorella* sp. HUC22-1, and those of the genus *Carboxydothermus.* Mixed cultures of two or more microorganisms may be used. Some examples of useful bacteria include *Acetogenium kivui, Acetobacterium woodii, Acetoanaerobium noterae, Butyrzbacterium methylotrophicum, Caldanaerobacter subterraneous, Caldanaerobacter subterraneous pacificus, Carboxydothermus hydrogenoformans, Clostridium aceticum, Clostridium acetobutylicum, Clostridium autoethanogenum* (DSM 19630 of DSMZ Germany), *Clostridium autoethanogenum* (DSM 10061 of DSMZ Germany), *Clostridium thermoaceticum, Eubacterium limosum, Clostridium ljungdahlii PETC* (ATTC 49587), *Clostridium ljungdahlii ERI2* (ATCC 55380), *Clostridium ljungdahlii C-01* (ATCC 55988), *Clostridium ljungdahlii O-52* (ATCC 55889), *Clostridium ultunense, Clostridium ragsdali P11* (ATCC BAA-622), *Alkalibaculum bacchi* CP11 (ATTC BAA-1772), *Clostridium coskatii, Clostridium carboxidivorans P7* (ATCC PTA-7827), *Geobacter sulfurreducens, Morrella thermacetica, Peptostreptococcus productus, Clostridium drakei,* and mixtures thereof. The fermentation process generally comprises contacting the product stream comprising CO, H₂ and CO₂ with the bacteria in the presence of a nutrient medium in a suitable reactor, for example a continuously stirred tank reactor (CSTR). It is clearly to be understood that the invention is not restricted to CSTRs and that other fermentation reactors can be used. Suitable temperatures and pressures are dependent on the bacteria and other process conditions used, but typical temperatures for the fermentation are between 25°C and 85°C, especially 35°C to 45°C and typical pressures are in the range atmospheric to 20 barg, preferably 2 to 17 barg. US 7 285 402 provides information about how to operate fermenters of use in the invention.

"Nutrient medium" is used generally to describe conventional bacterial growth media which contain vitamins and minerals sufficient to permit growth of a selected subject bacteria. Suitable nutrients are well-known, for example as described in US 7 285 402, WO 08/00 558, US 5 807 722, US 5 593 886 and US 5 821 111.

The agitation rate may be selected depending on the reaction vessel and robustness of the bacteria. In particular, the reaction mixture is generally agitated at a suitable rate to ensure adequate gas dispersion and substantial avoidance of agglomeration of dispersed gas bubbles whilst minimising damage to the bacterium cells caused by any moving parts e.g. stirrer tips.

In practice this usually means that for a larger unit agitated with a stirrer a smaller RPM (revolutions per minute) is used than for a corresponding smaller unit (for a fixed RPM, the tip speed of a larger agitator is faster than that of a smaller agitator). Speeds of 20 to 1000 RPM are typical, with larger units operating at the lower rates.

The residence time may also be selected depending on the particulars of the reaction, and in order to obtain the desired conversion. The residence time is usually in the range 5 seconds to 20 minutes, and most typically in the range 10 seconds to 5 minutes.

Generally the fermentation step produces a gas phase product stream comprising CO, H₂ and CO₂ and a liquid phase comprising ethanol, water, bacteria, nutrients and by-products such as acetic acid and higher alcohols. Of these products the greatest mass is water. The liquid is typically filtered to retain cells and any other solids present within the fermenter and to provide a filtered liquid phase for further processing. In addition, a portion of the fermenter broth containing bacterial cells is purged in order to maintain a healthy cell culture. It may be desirable to provide a plurality of fermenters.

The filtered liquid is then treated to concentrate the ethanol. For example the liquid phase could be distilled to give a water/ethanol mix. Typically the recovered mixture comprises 40-95wt% ethanol. The bottoms comprising water, residual nutrients and other materials such as acetic acid are cooled. The cooled bottoms can be recycled entirely or partially back to the fermenter optionally after treatment. The heat abstracted from the bottoms can be used elsewhere in the process. In some embodiments of the invention a plurality of distillation columns are used, preferred embodiments of this arrangement employ distillation columns that operate at different pressures. This means that the water/ethanol azeotrope boils at different temperatures in different columns and thus columns and operating pressures can be arranged such that the overheads from a first column act as a heat source for the reboiler of a second column. This multiple pairing of low pressure and high pressure columns allows ethanol separation at a very large scale and beyond that which may be practical to construct or transport as a single column. The rate of liquid feed to each column would be apportioned to achieve the desired optimum energy integration between the columns at their chosen operating pressures.

Components of the gas phase can be used in various ways. For example the gas phase can be recycled back to the reformer. Hydrogen could be separated from the mixture for example using a PSA. The separated hydrogen can be used either as a feedstock or as a fuel for example to heat a dehydration process to be described hereinafter or to fire a reformer.

In general it will be necessary to purge a portion of the gas phase to prevent accumulation of inert gases such as nitrogen and argon. The purge may be before or, preferably, after separation of components from the gas phase. Conveniently the purged gases are used as a fuel gas. This fuel gas can be used in other process steps such as for firing an externally fired reformer, or for raising steam in a fired steam boiler or in a fired furnace for heating the reactors of the dehydration stage.

The concentrated ethanol/water composition is then converted into ethylene in a dehydration process. Ethanol dehydration to ethylene is well known with references such as American Chemical Society Division of Fuel Chemistry 2007, 52, (2) p351-353 describing the historical context of use of fixed bed or fluid bed processes using alumina catalysts as well as exploration of an alternative option of Fluid Catalytic Cracking with Y-zeolite catalysts either as sole feed or as a co-feed in an oil refinery context to generate ethylene. Morschbacker in Journal of Macromolecular Science Part C: Polymer Reviews 2009,49, p79-84 also describes the manufacture of ethylene by the catalytic dehydration of ethanol derived from sugar cane ethanol and subsequent use for green polyethylene. A prominent comment by Morschbacker is that sugar derived ethanol suffers from price volatility and its conversion to ethylene is only economically viable at high oil prices. Thus a process which offers a price stable attractive route to ethylene would be desirable to the petrochemicals and olefins polymers industry. The process of the invention utilizing widely available gaseous carbonaceous feedstocks can provide such a route.

For example a part of the derived ethanol/water product could be further diluted with water typically to 50-90wt% ethanol. The water is preferably derived from other parts of the process for example as a co-product of the dehydration reaction. This feedstock is then vaporized and fed to a first dehydration reactor where the vaporized feedstock is passed over a catalyst such as γ-alumina to produce water and ethylene as primary products. In one embodiment, the additional water may be provided as steam and added after vaporisation of the ethanol/water product. The conversion proceeds with good selectivity, typically in excess of 85%, to ethylene. In at least some embodiments further vaporised ethanol product optionally without added water is added to the materials exiting the first reactor and the resulting mixture passed to a second dehydration reactor where the vaporized feedstock is passed over a catalyst such as γ-alumina to produce water and ethylene as primary products. This conversion proceeds with good selectivity again typically in excess of 85% to ethylene. In preferred embodiments of this variant the added ethanol is such that the ethanol/water ratio entering the second dehydration reactor is about the same as the ethanol/water ratio of the mixture entering the first dehydration reactor. In preferred embodiments the product leaving the first reactor plus additional feedstock is heated prior to entering the second dehydration reactor. In some embodiments the process is continued in like manner in a third and optionally further reactors.

In preferred embodiments the ethylene product is purified and recovered by distillation at low temperature generating a product suitable for polymerization to polyethylene. Ethylene suitable for polyethylene manufacturing requires very high purity, typically 99.90% with very low levels of any residual components such as hydrogen, methane, and ethane <2000ppm(vol) and total carbon oxides such as CO and CO₂ at approximately 1 ppnn(vol) level. In a most preferred embodiment recovered fractions of the reaction product stream which may comprise some ethylene arising from distillation separations, but may also comprise one or more of hydrogen, methane, ethane, propane, propylene and various ethers may be utilized in a co-located plant arrangement as feed for the reformer or less preferably for use as fuel gas.

In a further most preferred embodiment a portion of the recovered water from the reactor product stream is recovered, optionally treated, and utilised in the fermentation process to produce ethanol. Those skilled in the art will have no difficulty in deciding whether or not is necessary to treat the recovered water. Furthermore the skilled worker will have no difficulty in devising suitable treatment regimen such as filtering.

In preferred embodiments of the invention dehydration is carried out a pressure in the range of 0.1 to 3MN/m² preferably in the range of in the range of 0.5 to 2MN/m².

In other embodiments of the invention ethanol is converted into ethylene and water by passing over zeolite catalysts in a fixed bed or more preferably a fluid bed reactor system.

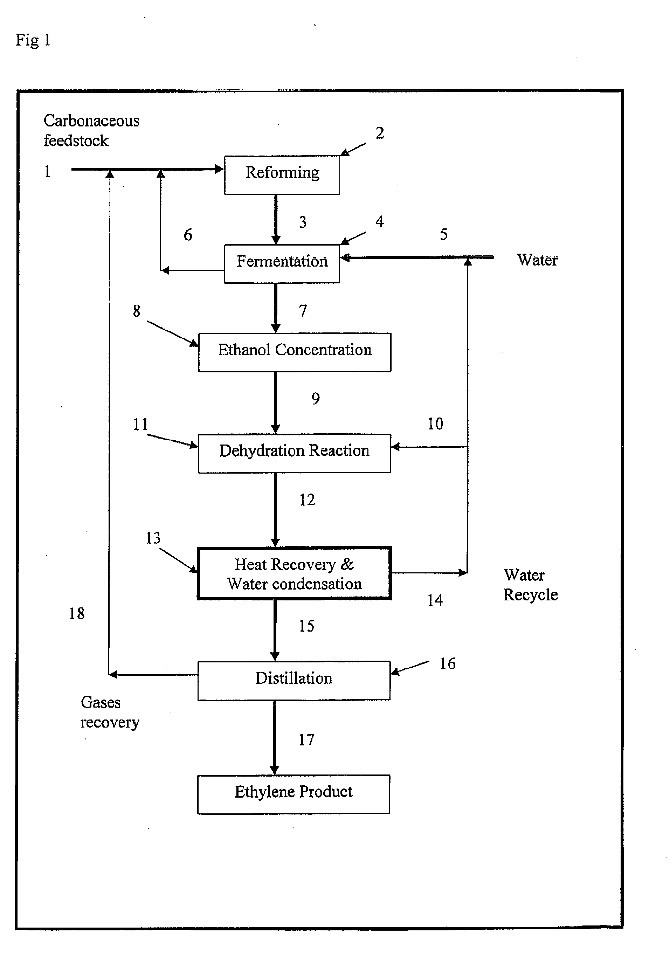

The process of the invention is illustrated in Fig 1. Carbonaceous feedstock is supplied from at least one source (not shown) via first conduit 1 to at least one reformer 2 as described herein. In the reformer or reformers 2 the carbonaceous feedstock is converted into a mixture comprising carbon dioxide, carbon monoxide and hydrogen. Other gases may be present also. The gases, generally after cooling in a cooler (not shown), are then passed via second conduit 3 into one or more fermenters 4. Water is added via third conduit 5. Nutrients and other materials may be included in the water or feed or added in other ways. As explained herein the fermenter contains a bacterial culture which transforms at least a part of the gas entering the fermenter into ethanol.

Typically material exiting the fermenter comprises two streams; a gas stream and a liquid stream. The gas stream can comprise one or more of carbon monoxide, carbon dioxide, hydrogen and inert gases such as nitrogen. Other materials such as water and ethanol may be present typically in lesser amount. Gas exiting the fermenter is typically recycled back into the reformer by fourth conduit 6. While not illustrated in Fig 1 a small portion of the gas exiting the fermenter may be bled off to prevent build up of inerts. This material can conveniently be used as a fuel in one of the other steps such as firing an externally fired reformer, or for heating the concentration or dehydration stages or for powering the refrigeration stage to be described herein.

The liquid phase comprises a solution of ethanol in water along with other materials such as other metabolites such as alcohols and acetic acid, nutrients, cell debris. Solids such as cell debris can be removed by filters (not shown). The liquid is then passed by fifth conduit 7 to concentrator 8. Concentrator 8 may comprise one or more distillation columns. An ethanol enriched stream exits the concentrator via sixth conduit 9 and an ethanol depleted stream (not shown) is rejected. As explained herein the ethanol depleted stream may be recycled back to the fermenter.

The ethanol enriched stream, optionally diluted with water, supplied via seventh conduit 10 is then dehydrated in dehydrator 11 as described herein. Dehydration is generally conducted at elevated temperature and gives rise principally to water and ethylene together with smaller amounts of diethyl ether and other material. The dehydrator product passes via eighth conduit 12 to heat recovery and water condensation stage 13. As the product is cooled water condenses and is, for example, run back to the fermentation stage by ninth conduit 14 optionally after further treatment. Heat can be recovered and used elsewhere in the system.

Typically the product is cooled yet further until the ethylene condenses into liquid form. The condensed ethylene then passes via tenth conduit 15 to distillation stage 16. Purified ethylene exits the distillation stage 16 via eleventh conduit 17 for further use. The purified ethylene is typically very cold and may be used to cool the ethylene for distillation at an earlier stage. An impurities stream is rejected from the distillation column and may pass via twelfth conduit 18 to the carbonaceous feedstock. Alternatively or additionally the rejected impurities may be used as fuel in other stages such as for firing an externally fired reformer, or for raising steam in a fired steam boiler or in a fired furnace for heating the reactors of the dehydration stage. Typically the cool impurities stream passes through a heat exchanger (not shown) to provide cooling within the refrigeration system that is used in the ethylene distillation step.

In other embodiments of the invention the ethanol is converted into ethylene and water by passing over other catalysts such as tungstosilicic heteropolyacid catalysts in a fixed bed reactor system. An example of such a process is described in WO 2008/138775.

The mixture exiting the dehydration step comprises water, ethylene and by-products such as diethyl ether as well as unreacted alcohol. The proportions of each component will depend on the type of catalyst being used and the exact reaction operating conditions of temperature and pressure. Ethylene has a very much lower boiling point than the water, diethyl ether or ethanol and is easily separated from them. These materials eg water, diethyl ether and ethanol can be recirculated to the dehydration reactors. Alternatively or additionally the diethyl ether can be removed for use in applications or used as a fuel in other steps of this process.

The ethylene is then subjected to an olefin derivatisation step. The nature of this step depends on the required product.

In one embodiment of the invention the ethylene is converted into polyethylene. In this process the ethylene is purified for example by cryogenic distillation to give an ethylene stream and an impurities stream which may include materials such as hydrogen, carbon monoxide, carbon dioxide, alkanes and higher alkenes as well as some ethylene. The impurities stream can then be passed to the reforming stage where it can be used as a fuel or, more preferably, as a feedstock for conversion to syngas and thence to ethanol.

The ethylene stream is then polymerized to give polyethylene such as high density polyethylene (HDPE), low density polyethylene (LDPE) and linear low density polyethylene (LLDPE) for example using known methods such as the Innovene S and Innovene G processes licensed by INEOS Technologies. In addition to polyethylene the process produces heat which can be used in other parts of the process. The polyethylene produced is typically degassed to remove volatiles such as hydrogen and unreacted ethylene from the polymer. The ethylene can be separated and recycled back into the polymerisation step. At least some of the hydrogen and or carbon containing material obtained by degassing can be recycled to the reforming step as either or both of feedstock or fuel for an externally fired reformer.

The ethylene can be converted into other materials. For example it can be converted into ethylbenzene by acid catalysed reaction with benzene. The ethylbenzene so formed can be dehydrogenated to give styrene and hydrogen. Typically this is done by passing it admixed with 10 to 15 times its volume of steam over an iron oxide catalyst. The heat and steam for the process can be, at least in part, obtained from other parts of the process. The obtained hydrogen and by products such as toluene can be used as fuels or as feedstock for the reformer.

In other embodiments the ethylene is converted into ethylene oxide. For example, this can be done by passing the ethylene and oxygen over silver on alumina catalyst. The products include heat, carbon dioxide and steam together with ethylene oxide. The ethylene oxide can be lead away for further use for example producing ethylene glycol, ethanolamine and crown ethers. The remaining products can be re-utilised for example as feedstock for the reformer.

In further embodiments of the invention the ethylene is converted into ethylene dichloride by reaction with chlorine in the presence of iron (III) chloride. The ethylene chloride may be used as is or it may be converted into other materials such as vinyl chloride monomer (VCM) for further elaboration.

In still other embodiments the ethylene is converted into vinyl chloride monomer (VCM) by reaction with hydrogen chloride and a molecular oxygen containing gas over a copper chloride catalyst. This reaction is strongly exothermic and the heat produced can be used in other parts of the process for example the ethanol dehydration step.

The process of the invention provides a carbon efficient route to olefin derivatives in large volume. While in principle biomass provides a useful potential source of biogas for reforming in practice sufficiently large amounts of biogas derived from biomass are not readily or widely available to compete on similar scale terms with current petrochemical routes to olefin derivatives. By way of example typical new world-scale olefins units produce of the order of 1 million metric tonnes of ethylene per year or more. Alternative strategies especially those for converting a natural gas methane containing feedstock into ethylene include
i) the so-called MTO methanol to olefins process in which methane is first converted into methanol and thence into olefins
ii) conversion of methane via the Fischer-Tropsch process into FT naphtha. The FT naphtha is then cracked in a conventional olefins furnace to yield ethylene and other olefins. Oil and Gas European Magazine March 1st 2010 (pages 44-47) compares processes producing ethylene from coal, biomass and natural gas compared with conventional steam cracking of naphtha or light gases (ethane+ propane +butane) and steam cracking of FT derived Naphtha. It appears from this article that only about 35-40% of the product mixture obtained by either steam cracking of FT naphtha or via the MTO process is ethylene. It is instructive to compare the efficiency of the process of the invention in producing ethylene with that of a process for producing ethylene using MTO or of Fischer Tropsch liquids to olefins.

In MTO the first step is production of methanol from natural gas. Haldor Topsoe A/S estimate a carbon loop efficiency of up to 95% (see http;//tinyurl.com/yabakx). The obtained methanol is then converted by the MTO process to give a mixture of olefins including ethylene, propylene and butenes. C5 hydrocarbons and fuel gas are also produced. According to Barger et al Table 2 on page 112 of a presentation entitled "Converting Natural Gas to Ethylene and Propylene using the UOP/Hydro MTO Process" from Advanced Catalytic Science and Technology Conference, Tokyo July 14-18, 2002 that ethylene is produced in equal mass as propylene at 882 metric te/d for a methanol feed of 5502 metric te/d giving 0.169 te ethylene yield per te methanol.

In the Fischer Tropsch process it is known that in a commercially worked process 9.34x 10⁶m³/d (330 x 10⁶ ft³/d) of lean methane rich gas produce 1430m³/d (9 000 barrels/d) of FT naphtha out of a total of 5400m³ (34000 barrels) of liquids. Since the density of ensuing FT naphtha is known to be about 700kgm⁻³ and the density of methane is known it is possible to deduce, knowing additionally that the upper yield of ethylene that can be expected from steam cracking of the naphtha is about 40%, that about 0.06 Te ethylene are produced per te methane.

### Comparative Example

Dry ethanol can be produced from a natural gas of composition as set out in Table 1 of our application number EP 10 190 527.1 using a combined reforming process as described in that application. The results, as detailed in Column 2 of Table 2, of the aforementioned application states a figure of 0.922 Te natural gas feedstock use per Te of ethanol product. The carbon dioxide emission figure also from Table 2, Column 2 is presented as 0.807 Te CO₂ /Te ethanol.

In the integrated process of the invention i.e. by conducting the reforming process, the fermentation process, the dehydration process and optionally at least one olefin derivatisation step in the same location (e.g. within 5 km preferably within 4 km of each other more preferably within 3km of each other) it is possible to utilise efficiently materials such as water and energy and by-products since waste from one step can be used in a further step. Where the processes are suitably co-located the recycling of water and waste carbonaceous fractions provides advantages particularly in locations where process water of suitable purity is scarce or expensive.

It is technically feasible for such derived waste streams to pump liquids or compress gaseous streams and transfer by means of pipelines to other co-located process steps without undue economic penalty however distance between process steps, flowrate, stream value and capital cost are all factors which determine economic viability for any particular separation distance being evaluated.

The benefits of integration can be illustrated by the following example.

### Example

Dry ethanol, when passed into a dehydration process comprising 3 reactors, containing γ-alumina catalyst, in series each operating adiabatically at 2.0 MN/m²(gauge) with feed ethanol and water fed to a 1^{st} reactor at 460°C with subsequent reheating of reaction product to 460°C to a 2^{nd} reactor with final ethanol addition and to a 3^{rd} reactor at 440°C. Ethanol is apportioned as 30% by mass to 1^{st} reactor and 35% each to subsequent reactors and first reactor ethanol feed is mixed with recycle water at a water to ethanol ratio of 3.04 to 1 by mass. A portion of the product stream from the final reactor is partially cooled against recycle preheat to 1^{st} reactor and then ethanol feed vaporisation with a further portion being used to raise steam before recombining and being further cooled to a point below which water is condensed from the product stream. A portion, as required, of this condensed water is recycled to the 1^{st} reactor vaporiser. Further cooling removes additional water from the gas stream. The condensed liquid fraction can subsequently be stripped to remove residual hydrocarbons for use as a fuel gas or reformer feed before its disposal or use in other parts of the combined process. Steam raised from cooling the reaction product is used within the process, in particular for reboiler of the condensed liquid stripping column to remove residual hydrocarbons and provide a higher purity water stream for use as a recycle stream to other steps or stages within the overall process. Steam generated is in excess of requirements and can be utilized as an energy source for other steps or stages within the overall process. Steam may also be utilized as an energy source for an optionally co-located ethylene derivative process.

Gas conditioning for removal of undesirable oxygenates from the stream is incorporated before a final molecular sieve drying step on the non condensed fraction to remove any further residual water from the stream. The dried ethylene enriched rich stream then passes into a cryogenic distillation column where it is cooled by a reflux stream at a temperature of less than -40°C of condensed pure ethylene liquid recovered from the top of a stripper column. The cryogenic distillation column reboiler provides heat to the base of the column allowing the separation of heavier than ethylene components to be removed as a liquid base stream with minimized loss of ethylene. An ethylene vapour stream leaves the top of the column with any lighter components and is passed via a condenser and a separation drum, from which a small light gases stream is removed, with the liquid being recovered and returned as a reflux stream to both the distillation and stripper columns. The purpose of the stripper column is to purify the ethylene and recover it as a liquid steam as a base stream with an overheads stream containing any light component being passed via the ethylene condenser. The heat source for the distillation reboiler and the stripping column reboiler plus the cooling for the stripper and distillation column condenser is provided from a propylene refrigeration system. The refrigeration system is designed such that the distillation column reboiler is heated by condensing and sub-cooling of a vapour propylene stream leaving a refrigeration compressor. This cooled liquid stream is then used as a heating fluid for the ethylene stripper column reboiler which cools the propylene further. The propylene is flashed to a lower pressure resulting in a very cold liquid stream which is used as the coolant for the ethylene condenser. The components recovered as the base liquid stream from the cryogenic distillation column includes any C2 and C3 alkanes plus any C3 alkenes and heavier components. Any light components present such as methane, hydrogen, carbon monoxide are removed from the columns in the condenser separating drum as an overheads stream. In this example, the C3 alkenes and heavier components are used as a component of the carbonaceous feedstock and the light gas fraction has its cold energy recovered in the refrigeration circuit before use as a component of the carbonaceous feedstock. The liquid ethylene derived from the stripping column base is a purified ethylene suitable for polymerization. The ethylene fraction recovered from the process as described above is 57.9% by mass of the ethanol fed to the reactors.

Where the condensed liquid fraction is stripped of hydrocarbons, the resulting stream can be partially recycled to the fermenter and/or optionally as water make-up to the reforming step a). In such instance, the portion of water available for recycle is determined as being 38.1% by mass of feed ethanol stream. The stream is comprised of predominately water (>98%) by mass with balance as small levels of impurities such as alcohols, ketones, organic acids and C4 hydrocarbons. Optionally, all of this stream can potentially be utilized as for a non-integrated scheme the water demand for steps a) and b) combined exceeds this available portion. This significant flow of water would have to be treated in a waste water treatment system and be disposed of as an effluent stream, if it were not recycled. The cost saving is therefore not just saving the cost of sourcing water, for example by desalination, but also saving the cost of disposing of waste water.

The ethylene depleted light and heavy carbonaceous fractions recovered during the ethylene recovery and purification step are determined, in this example, to be 1.9% as a portion by mass of the feed ethanol to the plant. This combined stream comprises ethylene, carbon monoxide, hydrogen, methane, ethane, propane, propylene and butene plus other minor C4 components.

This is a relatively small flow stream compared to the reforming section carbonaceous feed requirements and components such as these at low overall composition levels are not a significant problem for a pre-reformer catalyst in terms of coking, however portions of the stream may be optionally partially hydrogenated to minimize any undesirable reformer catalyst effects from occurring.

If we now consider use of the recovered ethylene depleted carbonaceous fractions from the dehydration step as a co feed to the reformer as used and described in the Comparative Example we find that for the same production rate of ethanol from the integrated process of the invention, the natural gas feedstock requirement is reduced. This also has the added benefit of reducing the overall carbon footprint for the ethanol producing step. These results are presented in Table 1.

**Table 1.**

| | Natural gas use Te/Te dry Ethanol | Carbon dioxide emission. Te/Te dry ethanol |
|---|---|---|
| Invention | 0.900 | 0.779 |
| Comparative Non -integrated ethanol step | 0.922 | 0.807 |
| Invention Performance improvement % | 2.4 | 3.5 |

Thus, the natural gas use can be calculated, giving approximately, 1.55 Te natural gas /Te ethylene as being required by the process of the invention. Comparison of this invention as an integrated process to a non integrated scheme and other different commercial methods for producing ethylene from natural gas is summarized in Table 2.

**Table 2**

| | Invention | Non-Integrated | MTO | Fischer Tropsch & Steam Cracking |
|---|---|---|---|---|
| Natural gas Te/ Te Ethylene | 1.55 | 1.59 | 3.12 | 16.8 |

It will therefore be apparent that the invention produces ethylene and ethylene derivatives far more efficiently in terms of mass of product per unit mass of methane containing feedstock than other processes and is enhanced over a non integrated ethanol step and dehydration step.

## Claims

1. A process for producing ethylene from a carbonaceous containing feedstock the process comprising the steps of
a) reforming the carbonaceous containing feedstock into a first product stream comprising carbon dioxide, carbon monoxide and hydrogen;
b) fermenting the first product stream with an acetogenic anaerobic bacteria to produce a second product stream comprising ethanol;
c) concentrating ethanol from the second product stream into a third product stream; and
d) dehydrating ethanol in the third product stream to produce a first, ethylene enriched, carbonaceous fraction, a second, ethylene depleted, carbonaceous fraction and water and
d1) recycling at least a part of the water, optionally after treatment, to step b) and/or to step a) and/or recycling at least a part of the second, ethylene depleted carbonaceous fraction as a feedstock to step a)

2. A process for producing an olefin derivative the process comprising producing ethylene by a process as claimed in claim 1 and
e) converting the ethylene into an olefin derivative.

3. A process as claimed in claim 1 wherein the steps a) to d), are performed within 5 km of each other.

4. A process as claimed in claim 2 wherein step e) comprises polymerizing ethylene to form polyethylene and/or converting ethylene to ethylene oxide and/or converting ethylene into ethylene dichloride, and/or converting ethylene into ethylbenzene and wherein optionally the ethylene oxide is further converted into ethylene glycol, ethanolamine or crown ethers.

5. A process as claimed in any one of the preceding claims wherein step d) is performed at a pressure in the range of 0.1 to 3MN/m².

6. A process as claimed in any one of the preceding claims wherein the dehydration to ethylene step d) is performed in a plurality of reactors.

7. A process as claimed in any one of the preceding claims wherein step d) uses a propylene refrigeration system in the recovery of the ethylene enriched carbonaceous fraction.

8. A process as claimed in any one of the preceding claims wherein the carbonaceous feedstock is at least partially obtained by anaerobic digestion of biomass.

9. A process as claimed in any one of the preceding claims wherein step c) comprises the step of distillation in a plurality of distillation stages arranged in parallel operating at different pressures such that distillate passing from a first distillation stage heats bottoms of a second distillation stage.

10. A process as claimed in any one of the preceding claims wherein surplus energy in the form of steam generated in the dehydration step d) of claim 1 is utilized in the ethanol recovery step c) or in other co-located ethylene derivative processes according to Claim 2 step e).

11. A process according to claim 2, wherein unreacted portions of the ethylene used in the derivative process are recovered, optionally treated, and used as a co-feed to the ethylene recovery cryogenic distillation stage of the ethanol dehydration step c).

12. A process for the production of ethanol according to claim 1 wherein the ethanol producing step b is performed in a plurality of biochemical reactors.

13. A process for the production of ethanol according to claim 1 wherein the carbonaceous feedstock reforming step a) comprises one or more, similar or different reforming reactors.

14. Apparatus for producing ethylene comprising
i) a reformer (2) for reforming a carbonaceous feedstock into a first product stream comprising carbon dioxide, carbon monoxide and hydrogen;
ii) a fermenter (4) for fermenting the first product stream into a second product stream comprising ethanol;
iii) means (8) for concentrating ethanol from the second product stream;
iv) a dehydrator (11) for dehydrating the ethanol to a first, ethylene enriched, carbonaceous fraction, a second, ethylene depleted, carbonaceous fraction and water; and either or both
v) means (18) for recycling at least a portion of the second, ethylene depleted, carbonaceous fraction to the reformer and
vi) means (14) for recycling at least a portion of the water to the fermenter (4) and/or reformer (2).

15. Apparatus as claimed in claim 14 further comprising vii) means for treating water recycled to the fermenter and/or reformer.

## Patentansprüche

1. Verfahren zum Herstellen von Ethylen aus einem kohlenstoffhaltigen Einsatzmaterial, wobei das Verfahren die Schritte umfasst
a) Reformieren des kohlenstoffhaltigen Einsatzmaterials zu einem ersten Produktstrom, der Kohlendioxid, Kohlenmonoxid und Wasserstoff umfasst;
b) Fermentieren des ersten Produktstroms mit einem acetogenen anaeroben Bakterium bzw. Bakterien, um einen zweiten Produktstrom zu erzeugen, der Ethanol umfasst;
c) Konzentrieren von Ethanol aus dem zweiten Produktstrom zu einem dritten Produktstrom; und
d) Dehydratisieren von Ethanol in dem dritten Produktstrom, um eine erste, mit Ethylen angereicherte, kohlenstoffhaltige Fraktion, eine zweite, an Ethylen verarmte, kohlenstoffhaltige Fraktion und Wasser zu erzeugen, und
d1) Rückführen von wenigstens einem Teil des Wassers, gegebenenfalls nach einer
Behandlung, zu Schritt b) und/oder zu Schritt a) und/oder Rückführen von wenigstens einem Teil der zweiten, an Ethylen verarmten, kohlenstoffhaltigen Fraktion als ein Einsatzmaterial zu Schritt a).

2. Verfahren zum Herstellen eines Olefinderivats, wobei das Verfahren das Herstellen von Ethylen durch ein Verfahren nach Anspruch 1 und
e) das Umwandeln des Ethylens in ein Olefinderivat umfasst.

3. Verfahren nach Anspruch 1, wobei die Schritte a) bis d) innerhalb von 5 km voneinander durchgeführt werden.

4. Verfahren nach Anspruch 2, wobei Schritt e) das Polymerisieren von Ethylen zum Bilden von Polyethylen und/oder das Umwandeln von Ethylen in Ethylenoxid und/oder das Umwandeln von Ethylen in Ethylendichlorid und/oder das Umwandeln von Ethylen in Ethylbenzol umfasst und wobei gegebenenfalls das Ethylenoxid weiter in Ethylenglycol, Ethanolamin oder Kronenether umgewandelt wird.

5. Verfahren nach einem der vorangehenden Ansprüche, wobei Schritt d) bei einem Druck im Bereich von 0,1 bis 3 MN/m² durchgeführt wird.

6. Verfahren nach einem der vorangehenden Ansprüche, wobei die Dehydratisierung zu Ethylen von Schritt d) in einer Mehrzahl von Reaktoren durchgeführt wird.

7. Verfahren nach einem der vorangehenden Ansprüche, wobei Schritt d) ein Propylen-Kühlsystem bei der Rückgewinnung der mit Ethylen angereicherten kohlenstoffhaltigen Fraktion verwendet.

8. Verfahren nach einem der vorangehenden Ansprüche, wobei das kohlenstoffhaltige Einsatzmaterial wenigstens teilweise durch anaeroben Abbau von Biomasse erhalten wird.

9. Verfahren nach einem der vorangehenden Ansprüche, wobei Schritt c) den Schritt der Destillation in einer Mehrzahl von Destillationsstufen umfasst, die parallel angeordnet sind und bei unterschiedlichen Drücken betrieben werden, so dass Destillat, das aus einer ersten Destillationsstufe geleitet wird, das Bodenprodukt einer zweiten Destillationsstufe erwärmt.

10. Verfahren nach einem der vorangehenden Ansprüche, wobei Überschussenergie in Form von Dampf, der in dem Dehydratisierungsschritt d) von Anspruch 1 erzeugt wird, in dem Ethanolrückgewinnungsschritt c) oder in anderen am gleichen Standort befindlichen Ethylenderivatverfahren gemäß Anspruch 2 Schritt e) verwendet wird.

11. Verfahren nach Anspruch 2, wobei nicht umgesetzte Teile des in dem Derivatverfahren verwendeten Ethylens zurückgewonnen, gegebenenfalls behandelt und als ein Co-Einsatzmaterial für die kryogene Destillationsstufe zur Ethylenrückgewinnung des Ethanoldehydratisierungsschritts c) verwendet werden.

12. Verfahren zur Herstellung von Ethanol gemäß Anspruch 1, wobei der Ethanolherstellungsschritt b in einer Mehrzahl von biochemischen Reaktionen durchgeführt wird.

13. Verfahren zur Herstellung von Ethanol gemäß Anspruch 1, wobei der Schritt a) zum Reformieren des kohlenstoffhaltigen Einsatzmaterials einen oder mehrere ähnliche oder unterschiedliche Reformierungsreaktoren umfasst.

14. Apparatur zum Herstellen von Ethylen umfassend
i) einen Reformer (2) zum Reformieren eines kohlenstoffhaltigen Einsatzmaterials zu einem ersten Produktstrom, der Kohlendioxid, Kohlenmonoxid und Wasserstoff umfasst;
ii) einen Fermenter (4) zum Fermentieren des ersten Produktstroms zu einem zweiten Produktstrom, der Ethanol umfasst;
iii) Mittel (8) zum Konzentrieren von Ethanol aus dem zweiten Produktstrom;
iv) einen Entwässerer (11) zum Dehydratisieren des Ethanols zu einer ersten, mit Ethylen angereicherten, kohlenstoffhaltigen Fraktion, einer zweiten, an Ethylen verarmten, kohlenstoffhaltigen Fraktion und Wasser; und entweder eines oder beide von
v) Mitteln (18) zum Rückführen von wenigstens einem Teil der zweiten, an Ethylen verarmten, kohlenstoffhaltigen Fraktion zu dem Reformer, und
vi) Mitteln (14) zum Rückführen von wenigstens einem Teil des Wassers zu dem Fermenter (4) und/oder Reformer (2).

15. Apparatur nach Anspruch 14, außerdem umfassend vii) Mittel zum Behandeln von zu dem Fermenter und/oder Reformer zurückgeführtem Wasser.

## Revendications

1. Procédé pour produire de l'éthylène à partir d'une charge carbonée, le procédé comprenant les étapes de
a) reformage de la charge carbonée en un premier courant de produit comprenant du dioxyde de carbone, du monoxyde de carbone et de l'hydrogène ;
b) fermentation du premier courant de produit avec une bactérie anaérobie acétogène pour produire un second courant de produit comprenant de l'éthanol ;
c) concentration de l'éthanol à partir du second courant de produit en un troisième courant de produit ; et
d) déshydratation de l'éthanol dans le troisième courant de produit pour produire une première fraction carbonée enrichie en éthylène, une seconde fraction carbonée appauvrie en éthylène et de l'eau et
d1) recyclage d'au moins une partie de l'eau, éventuellement après traitement, dans l'étape b) et/ou dans l'étape a) et/ou recyclage d'au moins une partie de la seconde fraction carbonée appauvrie en éthylène comme charge dans l'étape a).

2. Procédé pour produire un dérivé oléfinique, le procédé comprenant la production d'éthylène par un procédé selon la revendication 1 et
e) la conversion de l'éthylène en un dérivé oléfinique.

3. Procédé selon la revendication 1 où les étapes a) à d) sont accomplies dans des limites de 5 km l'une de l'autre.

4. Procédé selon la revendication 2 où l'étape e) comprend la polymérisation de l'éthylène pour former du polyéthylène et/ou la conversion de l'éthylène en oxyde d'éthylène et/ou la conversion de l'éthylène en dichlorure d'éthylène et/ou la conversion de l'éthylène en éthylbenzène et où éventuellement l'oxyde d'éthylène est converti encore en éthylèneglycol, éthanolamine ou éthers couronnes.

5. Procédé selon l'une quelconque des revendications précédentes où l'étape d) est accomplie à une pression dans la plage de 0,1 à 3 MN/m².

6. Procédé selon l'une quelconque des revendications précédentes où l'étape d) de déshydratation en éthylène est accomplie dans une pluralité de réacteurs.

7. Procédé selon l'une quelconque des revendications précédentes où l'étape d) utilise un système de réfrigération du propylène dans la récupération de la fraction carbonée enrichie en éthylène.

8. Procédé selon l'une quelconque des revendications précédentes où la charge carbonée est obtenue au moins partiellement par digestion anaérobie d'une biomasse.

9. Procédé selon l'une quelconque des revendications précédentes où l'étape c) comprend l'étape de distillation dans une pluralité de stades de distillation disposés en parallèle fonctionnant à des pressions différentes de sorte que le distillat passant d'un premier stade de distillation chauffe les fonds d'un second stade de distillation.

10. Procédé selon l'une quelconque des revendications précédentes où l'énergie en surplus sous forme de vapeur produite dans l'étape de déshydratation d) de la revendication 1 est utilisée dans l'étape de récupération de l'éthanol c) ou dans d'autres procédés de dérivés de l'éthylène au même site selon l'étape e) de la revendication 2.

11. Procédé selon la revendication 2, où les parties qui n'ont pas réagi de l'éthylène utilisé dans le procédé de dérivés sont récupérées, éventuellement traitées, et utilisées comme co-alimentation du stade de distillation cryogénique de récupération de l'éthylène de l'étape de déshydratation de l'éthanol c).

12. Procédé pour la production d'éthanol selon la revendication 1 où l'étape de production d'éthanol b) est accomplie dans une pluralité de réacteurs biochimiques.

13. Procédé pour la production d'éthanol selon la revendication 1 où l'étape de reformage de charge carbonée a) comprend un ou plusieurs réacteurs de reformage similaires ou différents.

14. Appareil pour produire de l'éthylène comprenant
i) un reformeur (2) pour reformer une charge carbonée en un premier courant de produit comprenant du dioxyde de carbone, du monoxyde de carbone et de l'hydrogène ;
ii) un fermenteur (4) pour fermenter le premier courant de produit en un second courant de produit comprenant de l'éthanol ;
iii) des moyens (8) pour concentrer l'éthanol provenant du second courant de produit ;
iv) un déshydrateur (11) pour déshydrater l'éthanol en une première fraction carbonée enrichie en éthylène, une seconde fraction carbonée appauvrie en éthylène et de l'eau ; et
v) des moyens (18) pour recycler au moins une partie de la seconde fraction carbonée appauvrie en éthylène dans le reformeur et/ou
vi) des moyens (14) pour recycler au moins une partie de l'eau dans le fermenteur (4) et/ou le reformeur (2).

15. Appareil selon la revendication 14 comprenant en outre vii) des moyens pour traiter l'eau recyclée dans le fermenteur et/ou le reformeur.
